(19) Europäisches Patentamt
European Patent Office
Office européen des brevets

(11) Veröffentlichungsnummer: **0 013 983**
**B1**

(12) # EUROPÄISCHE PATENTSCHRIFT

(45) Veröffentlichungstag der Patentschrift: **04.07.84**

(21) Anmeldenummer: **80100353.4**

(22) Anmeldetag: **23.01.80**

(51) Int. Cl.³: **A 61 K 31/00,**
C 07 C 49/727,
C 07 C 49/737,
C 07 C 69/21,
C 07 C 69/33,
C 07 C 69/40,
C 07 C 69/78,
C 07 C 101/08,
C 07 D 303/32,
C 07 D 493/08,
C 07 D 493/18
//(C07D493/08)

(54) **Antineoplastische Mittel auf Basis von nicht oder nur geringfügig irritierenden und/oder promovierenden Diterpenalkoholen und Derivaten davon und deren Herstellung.**

(30) Priorität: **23.01.79 DE 2902506**

(43) Veröffentlichungstag der Anmeldung:
**06.08.80 Patentblatt 80/16**

(45) Bekanntmachung des Hinweises auf die Patenterteilung:
**04.07.84 Patentblatt 84/27**

(84) Benannte Vertragsstaaten:
**AT BE CH FR GB IT LU NL SE**

(56) Entgegenhaltungen:

**CHEMICAL ABSTRACTS, Band 90, 1979, Seite 159, Zusammenfassung 17371c, Columbus, Ohio, USA, FIBACH, EITAN et al. "Heterogeneity of murine erythroleukemia cells with respect to tumor promotermediated inbition of cell differentiation"**

**Die Akte enthält technische Angaben, die nach dem Eingang der Anmeldung eingereicht wurden und die nicht in dieser Patentschrift enthalten sind.**

(73) Patentinhaber: **Stiftung Deutsches Krebsforschungszentrum**
**Im Neuenheimer Feld 280**
**D-6900 Heidelberg 1 (DE)**

(72) Erfinder: **Hecker, Erich, Prof. Dr.**
**Am Zapfenberg 38**
**D-6900 Heidelberg-Handschuhsheim (DE)**
Erfinder: **Osswald, Hans, Prof. Dr.**
**Dürerstrasse 20**
**D-6900 Heidelberg (DE)**
Erfinder: **Schmidt, Rainer, Dr.**
**Oberer Gaisbergweg 20**
**D-6900 Heidelberg (DE)**
Erfinder: **Opferkuch, Hans Josef, Dr.**
**Hölderlinstrasse 9**
**D-6901 Eppelheim (DE)**
Erfinder: **Sorg, Bernd, Dr.**
**Neuhöfer Strasse 30**
**D-6700 Ludwigshafen (DE)**
Erfinder: **Adolf, Walter, Dr.**
**Freiburger Strasse 24**
**D-6945 Hirschberg-Leutershausen (DE)**
Erfinder: **Youssef, Mahmoud**
**Kolbenzeil 22**
**D-6900 Heidelberg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 013 983 B1

(56) Entgegenhaltungen:

CHEMICAL ABSTRACTS, Band 89, 1978, Seiten 165, Zusammenfassung 192165q. Columbus, Ohio, USA, BRUNE, KAY et al. "Inflammatory, tumor initiating and promoting activities of polycyclic aromatic hydrocarbons and diterpene esters in mouse skin as compared with their prostaglandin releasing potency in vitro"

CHEMICAL ABSTRACTS, Band 89, 1978 Seite 150, Zusammenfassung 37755p Columbus, Ohio, USA WIGLER, MICHAEL et al. "Induction of plasminogen activator in cultured cells by macrocyclic plant diterpene esters and other agents related to tumor promotion"

CHEMICAL ABSTRACTS, Band 88, 1978, Seite 210, Zusammenfassung 184255h Columbus, Ohio, USA SCHMIDT, R.J. et al. "Production of inflammation by diterpene esters"

CHEMICAL ABSTRACTS, Band 83, 1975 Seite 621, Zusammenfassung 97619h Columbus, Ohio, USA, HERGENHAHN, M. et al. "Resiniferatoxin and other esters of novel polyfunctional diterpenes from Euphorbia resinifera and unispina"

CHEMICAL ABSTRACTS, Band 78, 1973 Seite 105, Zusammenfassung 12481f Columbus, Ohio, USA, HECKER, Erich et al. "New phorbol esters and related cocarcinogens"

CHEMICAL ABSTRACTS, Band 86, 1977, Seite 281, Zusammenfassung 68355a, Columbus, Ohio, USA, KOGISO, SHIGEFUMI et al. "Odoracin, a nematicidal constituent from Daphne odora"

EXPERIENTIA, Band 32, ausgeg. 15. September 1976, Basel, CH UPADHYAY, R.R. et al. "Tumor promoting constituent of Euphorbia serrata L. Latex.", Seiten 1196-97

CHEMICAL ABSTRACTS, Band 86, 1977, Seite 9, Zusammenfassung 138b, Columbus, Ohio, USA, FUJITA, EIICHI "Terpenoids. XXXVI. Biological and physiological acitivity. I. The antitumor and antibacterial activity of the Isodon diterpenoids"

(74) Vertreter: Deufel, Paul, Dr. et al,
Patentanwälte Müller-Boré, Deufel, Schön, Hertel
Lewald, Otto Isartorplatz 6 Postfach 26 02 47
D-8000 München 26 (DE)

**Beschreibung**

Die Erfindung betrifft neue antineoplastische Mittel großer therapeutischer Breite und guter Verträglichkeit, Verfahren zur Herstellung deren Wirkkomponenten bzw. Wirksubstanzen und die Verwendung der Wirksubstanzen.

Zur therapeutischen Anwendung gelangen antineoplastische Mittel, die dadurch gekennzeichnet sind, daß sie als antineoplastische Wirkkomponente nicht oder nur geringfügig irritierende bzw. nicht oder nur geringfügig promovierende polyfunktionelle Diterpene des Tiglian-, Daphnan-, Ingenan- und Lathyrantyps oder deren Derivate enthalten.

Der Ausdruck "nicht oder nur geringfügig irritierend" bzw. "nicht oder nur geringfügig promovierend" bedeutet eine irritierende oder promovierende Wirksamkeit von <1% der irritierenden Wirkung des 12-O-Tetradecanoyl-phorbol-13-acetats (TPA) bzw. der promovierenden oder irritierenden Wirkung von 9,10-Dimethyl-(12)-benzanthrazen (DNBA) und Crotonöl [E. Hecker, Zeitschrift für Krebsforschung 65, 325—333 (1963)]. Um die Ausdrucksweise zu vereinfachen, wird im folgenden nur noch von nicht irritierend bzw. nicht promovierend gesprochen, wobei jedoch auch die nur geringfügig irritierend bzw. nur geringfügig promovierenden Verbindungen gemäß obiger Definition mitumfaßt sein sollen. Der Begriff "irritierend" bzw. "nicht irritierend" ist wohl definiert. Diese Eigenschaft wird im standardisierten Irritationstest am Mäuseohr geprüft. Der Begriff "promovierend" bzw. "nicht promovierend" ist ebenfalls wohl definiert. Diese Eigenschaft wird im standardisierten Promotionstest an der Rückenhaut der Maus geprüft (siehe auch E. Hecker Methods in Cancer Research Vol. 6, 1971 S. 442 und 443).

Die im folgenden genannte Literatur, chemische Formeln und Aufzählung von Verbindungen sowie Versuchsprotokolle sind als Anlagen beigefügt.

Die chemische Struktur der irritierenden und teilweise promovierenden Prinzipien der Euphorbiaceen ist erst seit 1966 bekannt, als die Isolierung und Reindarstellung dieser Prinzipien, ihre Strukturaufklärung und die Charakterisierung ihrer biologischen Wirkung am hautreizenden Samenöl der Euphorbiacee Croton tiglium L. (Crotonöl) und ab 1968 bei zahlreichen anderen Euphorbiaceen und später auch bei Thymelaeaceen gelang 3)—5). Es handelt sich um Ester zahlreicher bis dahin unbekannter, tri- und tetrazyklischer, polyfunktioneller Diterpene, deren Strukturen sich auf die hypothetischen tri- bzw. tetrazyklischen Diterpenkohlenwasserstoffe Daphan (1), Tiglian (2) und Ingenan (3) zurückführen lassen (vgl. Formelschema Anlage 2). Die den irritierenden und teilweise promovierenden Estern zugrundeliegenden polyfunktionellen Diterpene, z.B. (1b), (2b) und (3b) (vgl. Anlage 2) zeigen keine irritierende bzw. promovierende Wirkung. Aus Euphorbiaceen wurden ferner auch Ester makrozyklischer, polyfunktioneller Diterpene isoliert, deren Strukturen sich von dem hypothetischen makrozyklischen Diterpenkohlenwasserstoff Lathyran (4) ableiten lassen.

Im Gegensatz zu einem Teil der von (1)—(3), also Daphnan, Tiglian und Ingenan abgeleiteten Ester zeigen jedoch die bisher gefundenen Ester des makrozyklischen Lathyrans ebenso wenig irritierende bzw. promovierende Wirkung, wie die zugrundeliegenden polyfunktionellen Diterpene, z.B. (4c) (vgl. Anlage 2). Die Struktur des Lathyrans bzw. der davon abgeleiteten Diterpene läßt vermuten, daß sie in engem Zusammenhang mit der Biogenese der irritierenden und teilweise promovierenden Prinzipien obiger Pflanzenfamilien stehen [6].

Die Erfindung umfaßt also im wesentlichen die Verwendung von nicht oder nur geringfügig irritierenden und nicht oder nur geringfügig promovierenden polyfunktionellen Diterpenen des Tiglian-, Daphnan-, Ingenan- und Lathyran- typs, ihrer Ester mit aliphatischen oder aromatischen (einschließlich aliphatisch/aromatischen) Säuren mit bis zu 20 C-Atomen, vorzugsweise bis zu 10 C-Atomen, sowie ihrer Äther bzw. Acetale mit Alkoholen bzw. geeigneten Carbonylverbindungen mit bis zu 20 C-Atomen, vorzugsweise mit bis zu 10 C-Atomen, als antineoplastische Wirkstoffe. Als bevorzugte Wirkkomponente(n) in antineoplastischen Mitteln dienen insbesondere Phorbol bzw. 4$\alpha$-Phorbol, Ingenol und Lathyrol und/oder — im Sinne einer guten Bioverfügbarkeit sowie Verträglichkeit — geeignete Derivate (wie nachfolgende beschrieben) davon. Sie ergeben zusammen mit üblichen pharmazeutischen Hilfsmitteln neue antineoplastische Mittel.

Um den Anforderungen an Bioverfügbarkeit und Verträglichkeit auf dem Hintergrund der physikalisch-chemischen Eigenschaften der antineoplastischen Wirkstoffe Rechnung zu tragen, werden für Arzneiformen bevorzugt nicht irritierende bzw. nicht promovierende saure Carbonsäureester bzw. deren Ammoniumsalze oder nicht irritierende bzw. nicht promovierende basische Ester von Aminosäuren bzw. Peptiden bzw. deren Ammoniumsalze verwendet.

Viele, wenn auch nicht alle der bisher bekannten irritierenden Diterpenester des Daphnan-, Tiglian- und Ingenantyps zeigen auch cocarcinogene oder bedingt krebsauslösende Wirkung [3)–5)]. Sie wurden im Initiations-/Promotions-/Progressions-Modell der Carcinogenese der Mäusehaut als Tumorpromotoren charakterisiert und zeigen in unabhängigen Versuchen weder mutagene [4), 5)] noch alkylierende Wirkung. Heute gelten sie als der Prototyp von Cocarcinogenen mit initiations-(oder tumor)-promovierender Wirkung [3), 4), 7)]. In der Ätiologie menschlicher Tumoren können sie — im Vergleich zu Solitärcarcinogenen (zur Terminologie vgl. loc.cit [8)] — die Rolle von Krebsrisikofaktoren zweiter Ordnung spielen [3), 4)].

Als typische Beispiele für irritierende und teilweise promovierende Diterpenester sind in Anlage 2 die Strukturen 9,13,14-ortho-Tetradekanoylresiniferonol (1a), 23-O-Tetradekanoyl-12-desoxyphorbol (2a) und 3-O-Hexadekanoylingenol (3a) wiedergegeben. Sie enthalten relativ wenig Sauerstoffunktionen und sind repräsentativ für das breite Spektrum irritierender und mehr oder weniger promovierender, polyfunktionelle Diterpenester. Ein Beispiel für nicht irritierende und nicht promovierende makrozyklische, polyfunktionelle Diterpenester ist das Lathyrol-3,5-diacetat-15-benzoat ("$L_3$") (4a). In den bisher bekannten Estern der Typen 1a—4a kann der Säureteil gesättigte bzw. ungesättigte aliphatische, aromatische und gemischt aliphatisch/aromatische Säuren enthalten. Der Diterpenteil ist nach der Art, Zahl und Verteilung der funktionellen Gruppen im jeweiligen Kohlenstoffskelett verschieden. Die den Estern 1a—4a entsprechenden typischen polyfunktionellen Diterpengrundalkohole — das Resinifernonol (1b), das 12-Desoxyphorbol (2b), das Ingenol (3b) und das Lathyrol (4b) sind in Anlage 2 zusammen mit weiteren, bei den nachfolgend beschriebenen Versuchen verwendeten typischen polyfunktionellen Diterpenen wiedergegeben.

Bei Untersuchungen, mit dem Ziel zu prüfen, ob irritierende aber nicht promovierende Diterpenester "anticarcinogene" bzw. antineoplastische Wirkung zeigen, wurde gefunden, daß Gemische von 13-Mono- bzw. 13,20-Diestern des 12-Desoxyphorbols (2b) mit verschiedenen kurzkettigen Fettsäuren bei lokaler Behandlung von Tumoren der Rückenhaut der Maus ausgeprägte antineoplastische Wirkung zeigen. Es sind jedoch keine therapeutisch anwendbaren Mittel, da sie toxisch (irritierend) sind. Es ist auch bekannt, daß gewisse Diterpenester der Strukturtypen 1—3, für die starke promovierende und irritierende Wirkung in Publikationen nachgewiesen wurde [3), 4)], bei den Leukämien P 388 und L 1210 antileukämogen wirken, wie die Übersicht der Anlage 3 zeigt. Auch solche Verbindungen eignen sich nicht für die Therapie, da zwar das Carcinom beseitigt würde, aber wegen der starken promovierenden und irritierenden Wirkung der Patient diese Behandlung nicht entragen würde.

Die durchweg vorhandene starke irritierende und/oder die daraus resultierende toxische Wirkung der bis dahin auf antineoplastische Wirkung geprüften Diterpenester läßt sie also für die klinische Anwendung als ungeeignet erscheinen. Es wurde aber überraschenderweise an mehreren transplantablen Tumoren, einschließlich der Leukämie P 388 gefunden, daß die antineoplastischen Qualitäten in der durch die Grundstrukturen 1—4 charakterisierten Stoffklasse anscheinend nicht mit der irritierenden Wirkung zusammenhängen. Vielmehr zeigen viele nicht irritierende bzw. nicht promovierende polyfunktionelle Diterpene der Strukturtypen 1—4 bessere antineoplastische Wirkung als die irritierenden bzw. teilweise promovierenden Diterpenester vom Typ 1a—3a. Dies ergibt sich aus dem Vergleich der chemotherapeutischen Effekte von irritierenden bzw. promovierenden Diterpenestern und von entsprechenden nicht irritierenden und nicht promovierenden, polyfunktionellen Diterpenalkoholen an transplantablen Tumoren, nämlich Sarkom 180 und Ehrlich-Ascites-Tumor (Anlage 4).

Nachdem bereits gezeigt wurde [3), 4)], daß irritierende Wirkung nicht notwendig mit promovierender Wirkung verknüpft ist, wurde nunmehr geprüft, ob die irritierenden Eigenschaften der Diterpenester mit deren antineoplastischer Wirkung ursächlich im Zusammenhang stehen. Zu den Versuchen, die in Anlage 4, Protokolle 1 und 2 zusammengefaßt sind, wurden die irritierenden und promovierenden [3), 4)] Diterpenester Simplexin (1d), TPA (2c) sowie Ingenol-3,20-dibenzoat (3c) und der irritierende, aber nicht promovierende (2,3)-Diterpenester Resiniferatoxin (1c) herangezogen. Als entsprechender nicht irritierender [3), 4)] polyfunktioneller Diterpenalkohol wurden Phorbol (2d) verwendet (alle Strukturen vgl. Anlage 2). Als Tumormodell wurden Sarkom 180 und das Ehrlich-Carcinom gewählt. Bei letzterem diente Endoxan (Cyclophosphamid) als positive Kontrolle.

Der Versuch mit dem soliden Sarkom 180 (Anlage 4, Protokoll 1) zeigte, daß sowohl die mehr oder weniger irritierenden und promovierenden Diterpenester TPA, Simplexin und Ingenol-3,20-dibenzoat als auch das nicht irritierende und, wie später noch erläutert, unter bestimmten Bedingungen nur schwach promovierende Phorbol zu Tumorhemmungen von mehr als 50% führen. Dabei wurde ebenso wie beim soliden hyperdiploiden Ehrlich-Carcinom zum Teil nur eine niedrigere Dosierung als 20% der DL 50 angewandt. Bei letzteren Versuchen wurden die irritierenden und/oder cocarcinogenen Diterpenester, wie Protokoll 2, Serie 3, 4 und 6 zeigt, zweimal wöchentlich gegeben, weil das Ehrlich-Carcinom im Vergleich zu dem in Protokoll 1 verwendeten Sarkom 180 durch Tumorhemmstoffe weniger beeinflussbar ist. Es zeigte sich, daß PTA (Serie 3) wie auch Resiniferatoxin (Serie 6) bei diesen Carcinomen weder eine kurative Wirkung noch eine signifikante Tumorhemmung (50%) erreichten. Das als positive Kontrolle zu Serie 2 verwendete Endoxan zeigte hinsichtlich Tumorhemmung und Heilungsrate (5 von 10 Tieren geheilt) den besten Effekt.

Bei allen Versuchsgruppen, die mit den irritierenden und/oder promovierenden Estern TPA, Simplexin, Ingenol-3,20-dibenzoat und Resiniferatoxin behandelt wurden, konnten jedoch nur durch Wechsel der subkutanen Applikationsstelle schwere Entzündungen der Haut verhindert werden. Trotz dieser Maßnahmen kam es zu entzündlichen Verhärtungen (Indurationen) an der Injektionsstelle, welche ca. acht Tage anhielten. Dagegen ergaben sich bei der subkutanen Anwendung von Phorbol keine Veränderungen an der Injektionsstelle. Es zeigt sich also eine geringe therapeutische Breite der irritierenden und/oder promovierenden Diterpenester, was sich insbesondere am Beispiel des Simplexins (Protokoll 2, Serie 4) zeigen läßt. Zwar konnte mit Simplexin in einer Dosierung von 2 $\times$ 2 mg/kg pro Woche eine mehr als 50%ige Tumorhemmung erreicht werden (Protokoll 2, Serie 4) und bei zwei Tieren trat eine Heilung ein, wobei von Heilung bei allen hier aufgeführten Versuchen dann gesprochen

wird, wenn ein völliger Tumorrückgang bis mindestens 90 Tage nach Versuchsende andauert, jedoch kam es in dieser Serie in der dritten Woche zu drei Todesfällen wegen toxischer Wirkungen des Simplexins.

Das Simplexin repräsentiert jedoch jenen Strukturtyp, der nach dem Ergebnis jüngerer Untersuchungen [3), 4)] und nach den Ergebnissen von Kupchan et al (vgl. Anlage 3) zum Teil hohe antileukämische Wirkung zeigt.

Im Gegensatz zu den irritierenden und/oder promovierenden Diterpenestern erwies sich jedoch Phorbol als besser verträglich. Die dreimalige orale oder subkutane Applikation von 40 mg/kg Phorbol (Protokoll 2, Serie 7, 8), also eine Gesamtdosis von 120 mg/kg Phorbol pro Woche, bringt etwa den gleichen Effekt wie die zweimalige subkutane oder intravenöse Applikation in einer Gesamtdosis von 80 mg/kg Phorbol pro Woche gemäß Serien 9 und 10 desselben Protokolls. Die orale Gabe von Phorbol (Serie 7) bewirkte trotz zweier geheilter Tiere beim übrigen Kollektiv keine signifikante Tumorhemmung, während in den weiteren Serien (8 — bis 10) neben zwei bzw. drei geheilten Tieren eine Tumorhemmung von mehr als 50% zu beobachten war.

Dies zeigt erstmals, daß der nicht bzw. nur geringfügig irritierende und nicht bzw. nur geringfügig promovierende Diterpenalkohol Phorbol überraschenderweise ausgeprägte antineoplastische Wirkung hat und gegenüber den irritierenden und/oder promovierenden Diterpenestern deutliche Vorteile besitzt, da die irritierenden Ester bei subkutaner Applikation zur Induration der Injektionsstelle führen und weder oral noch intravenös mehrfach appliziert werden können, weil sie hochtoxisch wirken.

Es hat sich weiter gezeigt, daß auch andere nicht irritierende und nicht promovierende Diterpenalkohole (3) der Strukturtypen 1—3 antineoplastische Wirkung haben, wie dies außer für Phorbol (2d) auch für 4$\alpha$-Phorbol (2f), das den Strukturtyp des 4$\alpha$-Tiglian repräsentiert, und 16-Hydroxy-ingenol (3e) gezeigt werden konnte.

Die Wirkung dieser Diterpenalkohole auf das intramuskulär implantierte Retothel-Sarkom wurde bei subkutaner Applikation mit der Wirkung von Endoxan verglichen (Anlage 5). Die Diterpenalkohole wurden in einer Dosierung von 2 × 80 mg/kg pro Woche gegeben. Dabei wurde jeweils nur eine Dosierung verwendet, während von Endoxan zwei Dosierungen getestet wurden.

In der angewandten Dosierung ergaben alle geprüften Diterpenalkohole eine deutliche, mit Endoxan vergleichbare Tumorhemmung. Dies steht im Gegensatz zur Ansicht, daß die Tumorhemmung auf die irritierende Wirkung von den erfindungsgemäßen Diterpenderivaten ähnlichen Verbindungen zurückzuführen sei. In diesem Zusammenhang sei auch darauf verwiesen, daß über die leukämogene Wirkung von Phorbol [9)—12)] widersprüchliche Arbeiten bekannt sind [13)], 4$\alpha$-Phorbol nicht leukämogen zu sein scheint und über eine schwach promovierende Wirkung von Phorbol, 4$\alpha$-Phorbol und 12-Desoxyphorbol berichtet wurde [10) 12) 14) 15)].

Demgemäß ergab die weitere Untersuchung nicht irritierender tri- und tetrazyklischer sowie biogenetisch verwandter makrozyklischer Diterpenderivate an der als Therapiemodell allgemein anerkannten Leukämie P 388 ebenfalls günstige Ergebnisse.

Gewisse höhere Ester der Diterpenalkohole vom Typus 1b—3b oder 1c, 2e und 3d, nämlich solche, die entweder aliphatische Säurereste mit niedrigerer Zahl von C-Atomen oder aromatische Säurereste enthalten, wie z.B. 2d, aber auch 4$\alpha$-Phorbol-12,13,20-triacetat, das — ebenso wie 4$\alpha$-Phorbol — den Strukturtyp des 4$\alpha$-Tiglians repräsentiert, wirken, ebenso wie andere mehrwertige Ester von Diterpenalkoholen mit Säuren niedriger Kohlenstoffzahl sowohl bei lokaler als auch bei systemischer Applikation, wenn überhaupt, viel weniger irritierend und promovierend als Mono- bzw. Diester vom Typ 1a—3a, die mindestens eine langkettige Fettsäure enthalten [3), 4)].

Auch die nicht irritierenden und nicht promovierenden Derivate des makrozyklischen Grundkohlenwasserstoffs Lathyran 4 (Anlage 2) eignen sich. Alle im Verlauf der Erfindung bekannt gewordenen polyfunktionellen Alkohole und Ester, die sich vom Lathyran bzw. vom 4$\beta$-Lathyran ableiten, wirken nicht irritierend.

Ähnlich wie für Phorbol und 4$\alpha$-Phorbol ist für Lathyrol und 6,17-Epoxylathyrol — bei systemischer Applikation — geringfügige promovierende Wirkung gezeigt worden. Sie wirker aber — anders als Phorbol — nicht leukämogen. Für ein makrozyklisches, polyfunktionelles Diterpen, das Jatrophon, das in Euphorbiaceen vorkommt und sich von dem von (4) verschiedenen, makrozyklischen Diterpen Jatrophan ableitet, ist antileukämische Wirkung nachgewiesen [6) 16) 17)].

Anlage 6 zeigt die Prüfung folgender Substanzen an der Leukämie P 388:

1. die polyzyklischen Diterpenalkohole 12-Desoxyphorbol (2b), Phorbol (2d) und 4$\alpha$-Phorbol (2f) als Vergleichssubstanzen, die in den anderen Tumormodellen bereits getestet wurden,

2. die entsprechenden mehrwertigen Ester Phorbol-12,13-20-triacetat, -tribenzoat (vgl. 2d) und 4$\alpha$-Phorbol-12,13-20-triacetat (vgl. 2f),

3. der makrozyklische Diterpenalkohol Lathyrol (4c),

4. die entsprechenden makrozyklischen Diterpenester $L_3$, das 6,17-Epoxylathyrol-3-phenylacetat-5,15-diacetat (4d) und Ingol-12-acetat (4b) sowie ein Gemisch der beiden ebenfalls makrozyklischen Diterpenester Myrsinol-(diacetat, heptanoat) und Myrsinol-(acetat, butyrat, valerat).

Weil bei dieser Leukämie Endoxan (Cyclophosphamid) erfahrungsgemäß in einmaliger Dosierung besser wirkt (Anlage 6, Serie 2, 3) als bei einer Gabe von zwei Dosen im Abstand einer Woche, wurde bei subkutaner Applikation teilweise auch ein unterschiedliches Dosierungsschema angewandt.

Hinsichtlich der Heilungsrate und der ILS (prozentuale Zunahme des Überlebens in der Versuchsserie im Vergleich zur Kontrollserie) erwies sich Phorbol in einer einmaligen Dosierung von 100 mg/kg vergleichbar mit dem günstigsten chemotherapeutischen Resultat mittels Endoxan (Serie 3, 4). In gleicher Weise zeigte 4$\alpha$-Phorbol in einer Dosis von 100 mg/kg (Serie 9) optimale Wirkung. Das 12-Desoxyphorbol (Serie 12, 13) erreichte in der verwendeten Dosierung 400 resp. 4 $\times$ 100 mg/kg zwar in der Heilungsrate noch ein bemerkenswertes Resultat, jedoch blieb der prozentuale Anstieg der Überlebenszeit gering. Phorbol-12,13-20-triacetat (Serie 14) zeigte schon in einer Dosierung von 6,5 mg/kg toxische Wirkungen. Phorbol-12,13-20-tribenzoat (Serie 23) erreichte bei einer Dosierung 100 mg/kg das Wirkungsoptimum, während sich die Gabe von 100 mg/kg an vier aufeinanderfolgenden Tagen (Serie 26) als geringer wirksam erwies. 4$\alpha$-Phorbol-12,13,20-triacetat (Serie 30—31) induzierte in seiner höchsten Dosierung eine chemotherapeutische Wirkung, die derjenigen von 120 mg/kg Endoxan (Cyclophosphamid) in dieser Versuchsreihe (Serie 4) gleichkam.

Lathyrol zeigte in einer Dosierung von 150 mg/kg (Serie 18) bei einer Heilungsrate von 5 Tieren eine signifikante Erhöhung der Überlebenszeit von 120%. Hingegen verdoppelte sich bei einer Dosierung von 4 $\times$ 75 mg/kg Lathyrol die Heilung (10 von 15 Mäusen geheilt), während die Überlebenszeit im Vergleich zur vorhergehenden Serie 18 abnahm. Die chemotherapeutische Wirkung von Ingol-12-acetat (Serie 20), das nur in einer Dosierung geprüft wurde, entspricht dem chemotherapeutischen Ergebnis von 80 mg/kg Endoxan (Serie 3) in dieser Versuchsreihe. Die Heilungsrate beim Myrsinolestergemisch erreichte ein Optimum, wenn an vier aufeinanderfolgenden Tagen jeweils 25 mg/kg Myrsinoltriester subkutan injiziert wurden.

Diese Ergebnisse an der Leukämie 388 bestätigen somit, daß nicht irritierende und nicht promovierende polyfunktionelle Diterpenalkohole der Strukturtypen 1—4 ausgeprägte antineoplastische Wirkung zeigen und daß auch nicht irritierende und nicht promovierende Ester entsprechender Diterpenalkohole mit nicht substituierten aliphatischen oder aromatischen Säuren niedriger bis mittlerer Anzahl von C-Atomen, insbesondere mit bis zu 20 C-Atomen, bzw. deren Äther mit Alkoholen, die obigen Säuren entsprechen, ebenfalls antineoplastische Wirkung zeigen. Ferner zeigten auch nicht irritierende Acetale von polyfunktionellen Diterpenalkoholen des Typs 1—4 mit Carbonylverbindungen, die obigen Säuren entsprechen, bei Sarkom 180 und der Leukämie P 388 ausgeprägte tumorhemmende Wirkung. Diese Ergebnisse beweisen, anders als aufgrund des Wissensstandes vermutet werden konnte (Anlage 3), daß die antineoplastischen Eigenschaften von Diterpenderivaten der Strukturtypen 1—4 nicht an deren irritierende bzw. promovierende Wirkung gebunden sind. Der Begriff Acetale, wie er hier verwendet wird, entspricht der neueren Nomenklatur der organischen Chemie und umfaßt nicht nur die früher als Acetale im engeren Sinn bezeichneten Verbindungen sondern auch Halbacetale sowie Ketale und Halbketale.

Die optimale Dosierung der Diterpenderivate hängt von der jeweiligen Pharmakokinetik der Substanz ab ist im einzelnen zu ermitteln, was leicht durch Reihenversuche möglich ist.

In den folgenden Tabellen 1 bis 5 sind besonders interessante Verbindungen zusammengefaßt, die sich für die erfindungsgemäße Verwendung eignen.

TABELLE 1

Polyfunktionelle, polyzyklische Diterpenalkohole des Tigliantyps aus Euphorbiaceen und Thymelaeaceen

| Grundalkohol | Oxygenierung | Pflanze | Ref. Nr. [+)] |
|---|---|---|---|
| 4-Desoxyphorbol | $O_5$ | Euphorbia tirucalli L. | 30 |
| 12-Desoxyphorbol | | E.triangularis Desf. | 39 |
| | | E. resinifera Berg.[a] | 58 |
| | | Pimelea prostrata Willd. | 125 |
| Phorbol | $O_6$ | C.tiglium L. | 49 |
| | | C.sparsiflorus Morong | 119 |
| | | C.oblongifolius Roxb. | 36 |
| | | E.tirucalli L.[b] | 30,31 |
| | | Sapium japonicum Pax und Hoffm. | 82 |
| 4-Desoxy-16-hydroxy-phorbol | | C. flavens L. | 55,120a |
| 12-Desoxy-16-hydroxy-phorbol | | E.cooperi N. E. Brown[b] | 38 |
| 16-Hydroxyphorbol | $O_7$ | Aleurites fordii Hemsl. | 84 |
| | | C. flavens L. | 55,120a |
| 12-Desoxy-5β-hydroxy-phorbol-6α,7α-oxid | | Hippomane mancinella L. | 2 |
| | | Pimelea prostrata Willd. | 18 |

[a] und viele andere Euphorbia-Spezies (vgl. 25).
[b] und einige andere Euphorbia-Spezies (vgl. 25).
[+)] alle Ziffern dieser Spalte und in den Fußnoten a und b beziehen sich auf das in Anlage 1, loc.cit. 4 enthaltene Literaturverzeichnis.

TABELLE 2

Polyfunktionelle, polyzyklische Diterpenalkohole des Daphnantyps aus Euphorbiaceen und Thymelaeaceen

| Grundalkohol | Oxygenierung | Pflanze | Ref. Nr.+) |
|---|---|---|---|
| Resiniferonol | $O_6$ | E.resinifera Berg | 59 |
| | | E.unispina N.E. Brown | 59 |
| | | E.poisonii Pax | 24 |
| | | H.mancinella L. | 4 |
| 5β-Hydroxyresini-feronol-6α,7α-oxid | $O_8$ | Hura crepitans L. | 97 |
| | | H.mancinella L. | 2 |
| | | Excoecaria agallocha L. | 83 |
| | | P.simplex F. Muell | 92,125 |
| | | P.prostrata Willd. | 125 |
| | | Daphne mezereum L. | 109 |
| 5β,12β-Dihydroxy-resiniferonol-6α,7α-oxid | $O_9$ | D.mezereum L. | 80 |
| | | Lasiosiphon burchelli Meisn. | 19 |
| | | Gnidia lamprantha Gilg | 70,72 |

+) alle Ziffern dieser Spalte beziehen sich auf das in Anlage 1, loc.cit. 4 enthaltene Literaturverzeichnis

TABELLE 3

Polyfunktionelle, polyzyklische Diterpensäuren des 1α-Alkyldaphnantyps aus Thymelaeaceen

| Grundsäure[a] | Oxygenierung | Pflanze | Ref. Nr.[+)] |
|---|---|---|---|
| 1,2,6,7-Tetrahydro-*21-methyl*-6α,7α-epoxy-*1α-nonyl-resiniferonol-30-säure* | $O_9$ | Daphnopsis racemosa Griseb | b |
| 1,2,6,7-Tetrahydro-5-hydroxy-*21-methyl*-6α,7α-epoxy-*1α-nonyl-resiniferonol-30-säure* | $O_{10}$ | P. simplex F. Muell | 126 |
| 3-Desoxo-1,2,6,7-tetrahydro-3,5-dihydroxy-*21-methyl*-6α,7α-epoxy-*1α-nonyl-resiniferonol-30-säure* | | P. prostrata Willd.<br>D. racemosa Griseb. | 126<br>126 |
| 1,2,6,7-Tetrahydro-5-hydroxy-6α,7α-epoxy-*1α-hexadecylresiniferonol-34,35-en-36-säure* | | Synaptolepis kirkii Oliv. | 126 |
| 1,2,6,7-Tetrahydro-5,29-dihydroxy-*21-methyl*-6α,7α-epoxy-*1α-nonyl-resiniferonol-30-säure* | $O_{11}$ | P.prostrata Willd. | 126 |
| 3-Desoxo-1,2,6,7-tetrahydro-3,5,18,29-tetrahydroxy-*21-methyl*-6α,7α-epoxy-*1α-nonylresiniferonol-30-säure* | $O_{12}$ | G. subcordata Meissn. | 73 |

[a] Die korrespondierende 1α-Alkyl-1,2-dihydroresiniferonol-α-säure ist jeweils unterstrichen.
[b] W. Adolf und E. Hecker, in Vorbereitung.
[+)] alle Ziffern dieser Spalte beziehen sich auf das in Anlage 1, loc.cit. 4 enthaltene Literaturverzeichnis.

TABELLE 4
Polyfunktionelle, polyzyklische Diterpenalkohole des Ingenantyps aus Euphorbiaceen

| Grundalkohol | Oxygenierung | Pflanze | Ref. Nr.[+)] |
|---|---|---|---|
| 5-Desoxyingenol | $O_4$ | E.myrsinites L.[a] | 25 |
| 20-Desoxyingenol | | E.kansui Liou. | 116 |
| | | E.helioscopia L. | b |
| Ingenol | $O_5$ | E.ingens E. Mey | 85 |
| | | E.lathyris L. | 1,3 |
| | | E.milii Ch.des Moulins | 114 |
| | | E.myrisnites L. | 25,91 |
| | | E.resinifera Berg[c] | 58 |
| | | E.tirucalli L. | 31 |
| | | E.esula L. | 71 |
| 20-Desoxy-16-hydroxy-ingenol | | E.marginata Pursh | 123 |
| 13-Hydroxyingenol | $O_6$ | E.kansui Liou | 117 |
| | | E.cyparissias L. | 86 |
| 16-Hydroxyingenol | | E.ingens E. Mey | 85 |
| | | E.lathyris L. | 1,3 |
| | | E.marginata Pursh | 123 |
| 13,19-Dihydroxy-ingenol | $O_7$ | E.cyparissias L. | 86 |

[a] und einige andere Euphorbia-Spezies (vgl. 25)
[b] H. Gotta und E. Hecker, unveröffentliche Ergebnisse
[c] und viele andere Euphorbia- und Elaeophorbia-Spezies (vgl. 25 und 6, 23, 115, 116, 118)
[+)] alle Ziffern dieser Spalte und in Fußnoten a und c beziehen sich auf das in Anlage 1, loc.cit. enthaltene Literaturverzeichnis.

TABELLE 5
Polyfunktionelle, makrozyklische Diterpenalkohole des Lathyrantyps aus Euphorbiaceen

| Grundalkohol | Oxygenierung | Pflanze | Ref. Nr.[+)] |
|---|---|---|---|
| Bertyadionol | $O_3$ | Bertya cuppressoidea | 39,40 |
| Jolkinol C | | Euphorbia jolkini Boiss. | 41 |
| Jolkinol D | | E.jolkini | 41 |
| Lathyrol | $O_4$ | E.lathyris L. | 33 |
| Jolkinol B | | E.jolkini | 41 |
| 7-Hydroxylathyrol | $O_5$ | E.lathyris | 38 |
| 6,17-Epoxy-lathyrol | | E.lathyris | 34,37 |
| | | Macaranga tanarius | 35 |
| | | Muell. Arg. | |
| Jolkinol A | | E. jolkini | 41 |
| Ingol | $O_6$ | E.ingens L. | 43 |
| | | E.resinifera Berg. | 44 |
| | | E.lactea | 45 |
| Myrsinol | | E.myrsinites L. | wurden nach der Anmeldung publiziert |
| Isomyrsinol | | E.myrsinites | |
| 18-Hydroxyingol | $O_7$ | E.marginata Pursh | 46 |
| | | E.unispina N.E. Brown | 47 |

[+)] alle Ziffern dieser Spalte beziehen sich auf das in Anlage 1, loc.cit. 6 enthaltene Literaturverzeichnis.

Allgemein kann gesagt werden, daß sich die nicht irritierenden und nicht promovierenden Diterpenalkohole der Strukturtypen 1—4, ihre nicht irritierenden und nicht promovierenden Ester mit nicht substituierten aliphatischen oder aromatischen Säuren mit bis zu 20 C-Atomen sowie ihre Äther bzw. Acetonide mit den Säuren analogen Alkoholen bzw. Ketonen mit bis zu 20 C-Atomen als neues antineoplastisches Wirkprinzip hervorragend eignen. Ihre Anwendung scheint im Vergleich zu antineoplastischen Alkylantien nicht nur ein geringeres Risiko im Hinblick auf "iatrogene Carcinogenese" zu bringen, sondern dürfte auch eine selektivere Schädigung von Tumorzellen neben normalen Zellen ermöglichen. Wegen der geringen Nebenwirkungen dieser Verbindungsklassen und der daraus resultierenden großen therapeutischen Breite dürfte das neue Wirkprinzip eine wesentliche Verbesserung der Chemotherapie von Krebskrankheiten bringen.

Zusammenfassend seien folgende nicht irritierende bzw. nicht promovierende polyfunktionellen Diterpene besonders zur Therapie von Tumoren hervorgehoben:

1. Tri- und tetrazyklische, polyfunktionelle Diterpen und Alkylditerpene der Typus 1—3, Daphnan, Tiglian und Ingenan die bis vier C=C-Doppelbindungen sowie 1 bis 15 Sauerstoffunktionen in beliebiger Position und Kombination enthalten, insbesondere die in den Tabellen 1—4 aufgeführten Tiglian-, Daphnan-, 1$\alpha$-Alkyldaphnan- sowie Ingenanderivate. Als typisches Beispiel für die Darstellung tri- und tetrazyklischer, polyfunktioneller Diterpene aus Pflanzenmaterial wird später die Darstellung des Tiglianabkömmlings Phorbol (vgl. 2d) aus dem Samenöl von Croton tiglium L. (Crotonöl) beschrieben,

1a. Ester der Diterpene und Alkyl-diterpene des Typus 1—3, wie sie oben in 1. gezeigt sind, mit einer oder mehreren gesättigten und/oder ungesättigten aliphatischen, aromatischen und aliphatisch/aromatischen Säuren mit bis zu 20 C-Atomen sowie deren mögliche Isomere bezüglich der Stellung der Esterfunktion(en) im Diterpenskelett,

1b. Äther der Diterpene und Alkyl-diterpene des Typus 1—3, wie sie oben in 1. gezeigt sind, mit einem oder mehreren gesättigten und ungesättigten aliphatischen, aromatischen und aliphatisch/aromatischen Alkoholen bzw. Phenolen mit bis zu 20 C-Atomen sowie deren mögliche Isomere bezüglich der Stellung der Ätherfunktion(en) im Diterpenskelett,

1c. Acetale der Diterpene und Alkyl-diterpene des Typus 1—3, wie sie oben in 1. gezeigt sind, mit einer oder mehreren gesättigten und ungesättigten aliphatischen, aromatischen und aliphatisch/aromatischen Carbonylverbindungen mit bis zu 20 C-Atomen sowie deren mögliche Isomere bezüglich der Stellung der Ätherfunktion(en) im Diterpenskelett,

1d. gemischte Ester/Äther bzw. Ester/Acetale, wie sie oben in 1a. und 1b. gezeigt sind, in beliebiger Kombination der Ester/Äther- bzw. Ester/Acetalfunktionen,

2. makrozyklische, polyfunktionelle Diterpene und Alkylditerpene des Typus 4, die bis vier C=C-Doppelbindungen sowie 1 bis 10 Sauerstoffunktionen in beliebiger Position und Kombination enthalten nämlich die in Tabelle 5 und Seite 42 aufgeführten bereits oben beschriebenen Lathyranderivate. Als typisches Beispiel für die Darstellung makrozyklischer, polyfunktioneller Diterpene aus Pflanzenmaterial wird später die Darstellung des Lathyranabkömmlings 6,17-Epoxylathyrol (vgl. 4d) aus dem Samenöl von Euphorbia lathyris beschrieben,

2a. Ester der Diterpene und Alkyl-diterpene des Typus 4, wie oben in 2.gezeigt, mit einer oder mehreren gesättigten und/oder ungesättigten aliphatischen, aromatischen und aliphatisch/aromatischen Säuren mit bis zu 20 C-Atomen sowie deren mögliche Isomere bezüglich der Stellung der Esterfunktion(en) im Diterpenskelett,

2b. Äther der Diterpene und Alkyl-diterpene des Typus 4, wie oben unter 2.gezeigt, mit einem oder mehreren gesättigten und ungesättigten aliphatischen, aromatischen und aliphatisch/aromatischen Alkoholen bzw. Phenolen mit bis zu 20 C-Atomen sowie deren mögliche Isomere bezüglich der Stellung der Ätherfunktion(en) im Diterpenskelett,

2c. Acetale der Diterpene und Alkyl-diterpene des Typus 1—3, wie oben unter 1. gezeigt, mit einer oder mehreren gesättigten und ungesättigten aliphatischen, aromatischen und aliphatisch/aromatischen Carbonylverbindungen mit bis zu 20 C-Atomen sowie deren mögliche Isomere bezüglich der Stellung der Ätherfunktion(en) im Diterpenskelett,

2d. gemische Ester/Ather bzw. Ester/Acetale, wie oben unter 2a. und 2b. gezeigt, in beliebiger Kombination der Ester/Äther- bzw. Ester/Acetalfunktionen,

2e. saure Ester oder basische Ester der Diterpene und Alkylditerpene des Typs 1—4

3. Salze der sauren oder basischen Ester nach 2e., insbesondere deren Ammoniumsalze.

Im folgenden werden bevorzugte Wirk-komponenten und ihre Herstellung beschrieben.

Um den Anforderungen an Bioverfügbarkeit und Verträglichkeit auf dem Hintergrund der physikalisch-chemischen Eigenschaften der antineoplastischen Wirkstoffe besonders gut Rechnung zu tragen, werden als antineoplastische Wirkkomponenten für Arzneiformen entweder nicht irritierende und nicht promovierende saure Carbonsäureester bzw. deren Ammoniumsalze verwendet

4.1. Saure Carbonsäureester bzw. deren Ammoniumsalze

Polyfunktionelle Diterpenalkohole, vorzugsweise Phorbol, 4$\alpha$-Phorbol und Ingenol, werden mit Dicarbonsäuren mit bis zu 20 Kohlenstoffatomen, vorzugsweise bis zu 10 Kohlenstoffatomen, z.B. Anhydriden von Dicarbonsäuren, vorzugsweise Bernsteinsäure, Glutarsäure oder Phthalsäure, in an sich bekannter Weise umgesetzt zu den

4.1.1. jeweiligen sauren 20-Monoestern, wobei die in der folgenden Tabelle 6 aufgeführten besonders bevorzugt sind;

TABELLE 6

Charakterisierung einiger nicht irritierender und nicht promovierender saurer Carbonsäureester des Phorbols, 4$\alpha$-Phorbols, Ingenols und Lathyrols

| Hemisuccinate | $R_f$ | Spektroskopische Daten UV $\lambda_{max}$ (nm) | $\varepsilon_{max}$ | IR $\nu_{OH}$ | $\nu_{CO}$ | $\nu_{C=C}$ | $ID_{50}$ (nMol) |
|---|---|---|---|---|---|---|---|
| Phorbol-20-hemisuccinat | 0,3 | 235 | 4 800 | breit | 1710 | 1625 | > 10 |
| 4$\alpha$-Phorbol-20-hemisuccinat | 0,2 | 237 | 7 200 | breit | 1734 | 1631 | >100 |
| Ingenol-20-hemisuccinat | 0,7 | 280 | 280 | breit | 1710 | 1640 | >100 |

4.1.2. den jeweiligen sauren 12,13-bzw. 3,5-Di-oder entsprechenden sauren Monoestern von vorzugsweise Phorbol, 4$\alpha$-Phorbol, Ingenol und Lathyrol, nämlich insbesondere

Phorbol-12,13-di-hemisuccinat

4$\alpha$-Phorbol-12,13-di-hemisuccinat

Ingenol-3,5-di-hemisuccinat

Lathyrol-3,5-di-hemisuccinat

Phorbol-12-hemisuccinat
Phorbol-13-hemisuccinat
4α-Phorbol-12-hemisuccinat
4α-Phorbol-13-hemisuccinat
Ingenol-3-hemisuccinat
Ingenol-5-hemisuccinat
Lathyrol-3-hemisuccinat
Lathyrol-5-hemisuccinat

4.1.3. den jeweiligen 4,9-Di- bzw. 4-Mono- bzw. 15-Monoestern von vorzugsweise Phorbol, 4α-Phorbol, Ingenol und Lathyrol, nämlich insbesondere

Phorbol-4,9-di-hemisuccinat
4α-Phorbol-4,9-di-hemisuccinat

Phorbol-4-hemisuccinat
Phorbol-9-hemisuccinat
4α-Phorbol-4-hemisuccinat
4α-Phorbol-9-hemisuccinat
Ingenol-4-hemisuccinat
Lathyrol-15-hemisuccinat

Als antineoplastische Wirkkomponente werden entweder die vorerwähnten sauren Ester verwendet oder im Sinne einer weiteren Verbesserung der Löslichkeit sowie der Neutralisierung der sauren Komponente, was zu einer Verbesserung der Gewebever-träglichkeit führt, deren Salze mit Basen, vorzugsweise mit pharmakologisch gut verträglichen Aminen wie z.B. Aminoalkoholen oder Morpholin.

Ein Herstellungsbeispiel wird später noch aufgeführt.

4.2 Basische Ester mit Aminosäuren oder Peptiden bzw. deren Ammoniumsalze

Die polyfunktionellen Diterpenalkohole Phorbol, 4α-Phorbol und Ingenol werden mit Aminosäuren oder geeignet geschützten Derivaten davon oder mit Peptiden oder Derivaten davon umgesetzt zu

4.2.1. den jeweiligen 20-Monoestern, von denen bevorzugte Biespiele in der folgenden Tabelle 7 aufgeführt sind.

TABELLE 7

Charakterisierung einiger nicht irritierender und nicht promovierender Ester des Phorbols, 4α-Phorbols, Ingenols und Lathyrols mit Alanin bzw. β-Alanin

| Ester | $R_f$ | Spektroskopische Daten UV $\lambda_{max}$ (nm) | UV $\varepsilon_{max}$ | IR $\nu_{OH}$ | IR $\nu_{CO}$ | IR $\nu_{C=C}$ | $ID_{50}$ (nMol) |
|---|---|---|---|---|---|---|---|
| 20-O-Alanylphorbol | 0,30 | 235 | 4 800 | breit | 1710 | 1625 | >100 |
| 20-O-β-Alanylphorbol | 0,25 | 235 | 4 800 | breit | 1710 | 1625 | >10 |
| 20-O-Alanyl-4α-phorbol | 0,20 | 237 | 7 200 | breit | 1734 | 1630 | >10 |
| 20-O-β-Alanyl-4α-phorbol | 0,15 | 237 | 7 200 | breit | 1734 | 1630 | >10 |
| 20-O-Alanylingenol | 0,70 | 280 | 280 | breit | 1710 | 1640 | >100 |
| 20-O-β-Alanylingenol | 0,65 | 281 | 280 | breit | 1710 | 1640 | >10 |

4.2.2. den jeweiligen 12,13-Di-bzw. 3,5-Di- oder entsprechenden Monoestern von vorzugsweise Phorbol, 4α-Phorbol, Ingenol und Lathyrol, nämlich insbesondere

12,13-Di-O-alanylphorbol
12,13-Di-O-β-alanylphorbol

12,13-Di-O-alanyl-4$\alpha$-phorbol
12,13-Di-O-$\beta$-alanyl-4$\alpha$-phorbol
3,5-Di-O-alanylingenol
3,5-Di-O-$\beta$-alanylingenol
3,5-Di-O-alanyllathyrol
3,5-Di-O-$\beta$-alanyllathyrol

12-O-Alanylphorbol
12-O-$\beta$-Alanylphorbol
13-O-Alanylphorbol
13-O-$\beta$-Alanylphorbol
12-O-Alanyl-4$\alpha$-phorbol
12-O-$\beta$-Alanyl-4$\alpha$-phorbol
13-O-Alanyl-4$\alpha$-phorbol
13-O-$\beta$-Alanyl-4$\alpha$-phorbol
3-O-Alanylingenol
3-O-$\beta$-Alanylingenol
5-O-Alanylingenol
5-O-$\beta$-Alanylingenol
3-O-Alanyllathyrol
3-O-$\beta$-Alanyllathyrol
5-O-Alanyllathyrol
5-O-$\beta$-Alanyllathyrol

4.2.3. den jeweiligen 4,9-Di- bzw. 4-Mono- und 15-Monoestern von vorzugsweise Phorbol, 4$\alpha$-Phorbol, Ingenol und Lathyrol, nämlich insbesondere

4,9-Di-O-alanylphorbol
4,9-Di-O-$\beta$-alanylphorbol
4,9-Di-O-alanyl-4$\alpha$-phorbol
4,9-Di-O-$\beta$-alanyl-4$\alpha$-phorbol

4-O-Alanylphorbol
4-O-$\beta$-Alanylphorbol
9-O-Alanylphorbol
9-O-$\beta$-Alanylphorbol
4-O-Alanyl-4$\alpha$-phorbol
4-O-$\beta$-Alanyl-4$\alpha$-phorbol
9-O-Alanyl-4$\alpha$-phorbol
9-O-$\beta$-Alanyl-4$\alpha$-phorbol
4-O-Alanylingenol
4-O-$\beta$-Alanylingenol
15-O-Alanyllathyrol
15-O-$\beta$-Alanyllathyrol

In analoger Weise werden auch Ester mit Peptiden, vorzugsweise solchen, die physiologisch vorkommen, wie etwa Gluathion, und deren Salze mit pharmakologisch gut verträglichen anorganischen und organischen Säuren, verwendet.

Als antineoplastische Komponenten werden entweder die vorerwähnten basischen Aminosäureester bzw. Peptidester oder- im Sinne einer weiteren Verbesserung der Löslichkeit sowie der Neutralisierung der basischen Komponente, was zu einer Verbesserung der Gewebeverträglichkeit führt, — deren Salze mit pharmakologisch gut verträglichen anorganischen bzw. organischen Säuren, verwendet.

Die Anwendung der oben unter 4.1 und 4.2 beschriebenen, neuen Derivate polyfunktioneller Diterpene als antineoplastische Wirkkomponenten entsprechender Mittel hat den Vorzug der besseren Löslichkeit und Gewebeverträglichkeit gegenüber z.B. Phorbol, 4$\alpha$-Phorbol, Ingenol und Lathyrol.

Ein Herstellungsbeispiel wird ebenfalls später noch angegeben.

5. Arzneiformen

Die Arzneiformen, die zur Anwendung gelangen, orientieren sich in üblicher Weise an der beabsichtigten Applikationsweise und an der Höhe der zu wählenden Einzeldosis. Dabei ist insbesondere zu beachten, daß die Diterpenalkohole und ihre Derivate grundsätzlich als instabil (z.B. gegenüber Sauerstoff, Säuren, Alkali und Licht) anzusehen sind. Es ist daher bei allen Arzneiformen zu empfehlen, die antineoplastische Komponente in fester Form (Arzneiform: Tablette oder Kapsel; für parenteral zu applizierende Arzneiformen als Trockensubstanz in Ampullenform) tiefgekühlt vorrätig zu halten. Für

parenterale Applikation ist dann die Lösung oder Suspension jeweils frisch zu bereiten. Eine Übersicht gibt die folgende Tabelle 8, die sich an W.A. Ritschel, Angewandte Biopharmazie, Wiss. Verlagsgesellschaft Stuttgart, 1973 anlehnt.

TABELLE 8

Arzneiformen für antineoplastische Wirkkomponenten auf der Basis der beanspruchten polyfunktionellen Diterpene bzw. -derivate.

| Applikationsart/Verabreichung | | Arzneiform |
|---|---|---|
| peroral | p.o. | Tablette [a] |
| | | Kapsel [a] |
| parenteral | i.v. | hydrophile Lösung [b] |
| | i.m. | hydrophile lösung [c] oder Susp. |
| | i.m. | ölige Lösung oder Susp. [d] |

[a] Mit üblichen Hilfsstoffen (vgl. Ritschel, Tab. 23.3, S. 430), magensaftresistant durch Überszug mit filmbildenden Materialien (Schutz der Wirkstoffe vor möglichen Veränderungen durch stark saures Milieu des Magensaftes), Beispiele vgl. Ritschel, ab. 22.4, S. 401.
[b] Als Lösungsmittel können Polyäthylenglykol und Äthanol, als Solubilisierungsmittel Cremophor EL® vorgeschlagen werden; vgl. Ritschel, Tab. 24.9, S. 448 sowie Tab. 24.6, S. 446 und Tab. 24.8, S. 447.
[c] Als Lösungsmittel dient z.B. Polyäthylenglykol
[d] Als Vehikel dient z.B. Sesamöl, Isopropylmyristat o.ä.; vgl. Ritschel, Tab. 25.2, S. 463 und S. 461 ff.

Die folgenden Beispiele beschrieben die Herstellung saurer Carbonsäureester und deren Ammoniumsalze sowie die Herstellung basischer Ester mit Aminosäuren oder Peptiden und deren Ammoniumsalze und schließlich in der Zusammenfassung unter 1 und 2 erwähnt die Darstellung des Tiglian-Abkömmlings Phorbol sowie des Lathyran-Abkömmlings 6,17-Epoxylathyrol.

Herstellungsbeispiel 1

Herstellung von sauren Carbonsäureestern bzw. deren Ammoniumsalze.

Jeweils 1 mMol Phorbol, 4$\alpha$-Phorbol, Ingenol oder Lathyrol werden in 30 ml wasserfreiem Lösungsmittel (z.B. Methylenchlorid oder Tetrahydrofuran) suspendiert bzw. gelöst, mit 1 mMol Bernsteinsäure-anhydrid und 2 mMol wasserfreiem Pyridin versetzt und bei Raumtemperatur oder leichtem Erwärmen stehen gelassen bzw. (bei Suspensionen) gerührt. Die Monoveresterung ist nach 1 h abgeschlossen, wovon man sich durch einen dünnschichtchromatographischen Test überzeugt. Zur Aufarbeitung wird der Ansatz in 2n Sodalösung gegossen und mit Äther 2 bis 3 mal ausgeschüttelt. Nach Ansäuern der Sodalösung wird das Hemisuccinat mit Äthylacetat extrahiert. Die Hemisuccinate sind nicht kristallisierende Harze. Zur Reinheitsprüfung wird Dünnschichtchromatographie und zur Charakterisierung der $R_f$-Wert und die Anfärbung (Vanilin/Schwefelsäure), UV- und die IR-Daten sowie die $ID_{50}$ herangezogen (vgl. Tabelle 6).

In analoger Weise stellt man die unter 4.1.2 und 4.1.3 angegebenen Ester her, wobei bei Phorbol, 4$\alpha$-Phorbol und Ingenol zweckmäßig jeweils von den 20-Triphenylmethyläthern ausgegangen wird. Diese Schutzgruppe läßt sich nach der Umsetzung der Triphenylmethyläther mit dem Anhydrid und Isolierung der entsprechenden Dihemisuccinat-triphenylmethyläther bzw. Hemisuccinat-triphenylmethyläther in bekannter Weise selektiv wieder abspalten. Die so erhaltenen Succinate werden auf Reinheit durch Dünnschichtchromatographie geprüft und durch $R_f$-Wert, die UV- und die IR-Daten sowie die $ID_{50}$ am Mäuseohr charakterisiert.

Zur Herstellung der Salze werden die Succinate z.B. in Dioxan gelöst und mit der stöchiometrischen Menge der jeweiligen Base versetzt. Das ausfallende Salze wird abgesaugt und kann in der vorliegenden kristallinen Form direkt der Verwendung zugeführt werden.

Hergestellungsbeispiel 2

Herstellung von basischen Estern mit Amnosäuren oder Peptiden.

Jeweils 1 mMol Phorbol, 4$\alpha$-Phorbol, Ingenol oder Lathyrol werden in 50 ml wasserfreien Lösungsmittel (z.B. Tetrahydrofuran oder Dimethylsulfoxid) suspendiert bzw. gelöst, mit 1,3 mMol Alanin, $\beta$-Alanin oder Glutathion sowie 2 mMol Carbidiimid und 2 mMol Pyridin versetzt und bei Raumtemperatur stehen gelassen bzw. (bei Suspensionen) gerührt. Die Monoveresterung ist nach 30 min abgeschlossen, wovon man sich durch einen dünnschichtchromatographischen Test überzeugt. Zur Aufarbeitung wird der Ansatz in 2 n HCl gegossen und mit Äther 2 bis 3 mal ausgeschüttelt. Nach

Versetzen der HCl-Lösung mit 2 n Sodalösung bis pH 10 erreicht ist, wird der basische Aminosäure- bzw. Peptid-ester mit Butanol extrahiert. Die Ester kristallisieren nicht. Zur Reinheitsprüfung werden Dünnschichtchromatographie und zur Charakterisierung der $R_f$-Wert (Vanilin/Schwefelsäure), die UV- und die IR-Daten sowie die $ID_{50}$ herangezogen (vgl. Tabelle 7).

In analoger Weise stellt man die in 4.2.2 und 4.2.3 angegebenen Ester her, wobei bei Phorbol, 4$\alpha$-Phorbol und Ingenol zweckmäßig von den 20-Triphenylmethyläthern ausgegangen werden kann. Diese Schutzgruppe läßt sich nach der Umsetzung der Triphenylmethyläther mit der Aminosäure bzw. dem Peptid zu den entsprechenden Ester-triphenylmethyläthern in bekannter Weise selektiv wieder abspalten. Die 50 erhaltenen Aminosäure- bzw. Peptidester werden auf Reinheit durch Dünnschichtchromatographie geprüft. Man charakterisiert sie mittels $R_f$-Wert, UV- und IR-Daten sowie mit der $ID_{50}$ am Mäuseohr.

Zur Herstellung der Salze werden die Aminosäure- bzw. Peptidester z.B. in Dioxan gelöst und mit der stöchiometrischen Menge der jeweiligen Säure versetzt. Das ausfallende Salze wird abgesaugt und kann in der vorliegenden kristallinen Form direkt der Verwendung zugeführt werden.

## Herstellungsbeispiel 3

Darstellung des tetrazyklischen, polyfunktionellen Diterpens Phorbol aus Crotonöl

500 g des auf übliche Weise aus dem Samen von Croton tiglium L. gewonnenen Crotonöls werden mit einer Lösung von 55 g $Ba(OH)_2.8\ H_2O$ in 2,25 l Methanol 10 bis 12 h lang unter Stickstoff geschüttelt. Man filtriert von den ausgefallenen Bariumseifen ab und engt das Filtrat am Rotationsverdampfer bei 40°C Badtemperatur ein, bis kein Methanol mehr übergeht. Der ölige Rückstand wird mit 2 1 Wasser aufgenommen und das Gemisch zweimal mit je 500 ml Äther extrahiert. Die verbleibende Wasserphase (in der das Phorbol enthalten ist) wird mit 2 N Schwefelsäure auf pH 5 eingestellt. Nach Zugabe von 40 ml gesättigter Natriumsulfatlösung wird die Mischung 12 h bei 4°C aufbewahrt. Das ausgefallene Bariumsulfat wird dann abgesaugt, die Lösung mit 2 N Natronlauge auf pH 7 eingestellt und nacheinander mit je 500 ml Essigsäureäthylester und Äther extrahiert. Die wässrige Phase wird am Rotationsverdampfer bei 45°C eingeengt, bis kein Wasser mehr übergeht. Man versetzt den viskosen Rückstand mit 100 ml Äthanol und filtriert das ausgefallene Natriumsulfat ab. Der Natriumsulfat-Rückstand wird mit warmem Äthanol so oft extrahiert, bis eine Probe beim Kochen mit 1 ml konz. Salzsäure keine (oder nur noch eine sehr schwache) Rotfärbung zeigt ("Phorbolreaktion"). Die vereinigten Filtrate werden auf 50 bis 60 ml eingeengt und die viskose Lösung bei 4°C aufbewahrt. Phorbol kristallisiert aus dieser Lösung meist spontan; ist das nicht der Fall, so kann die Lösung zu viskos sein und muß dann mit wenig Äthanol verdünnt werden. Nach 4 Wochen werden die Kristalle abgesaugt, auf Tonplatten ausgebreitet und so einige Tage bei 4°C unter Stickstoff aufbewahrt. Auf diese Weise erhält man 5,5 bis 6,1 g rein weißes Phorbol-$C_2H_5OH$ ("Alkoholphorbol"). Das Solvat ist instabil und wird in Phorbol ("Wasserphorbol") übergeführt; dazu werden z.B. 5,8 g Phorbol.$C_2H_5Oh$ in 100 ml Wasser bei 60°C gelöst. Die Lösung wird im rotationsverdampfer bei 60°C eingeengt, bis sich Kristalle abschieden. Man hält diese Präparation 1 Woche lang bei 4°C unter Stickstoff und saugt dann die abgeschiedenen Kristalle ab (z.B. 4,4 g Phorbol, Fp. 250—1°C, Zers.).

## Herstellungsbeispiel 4

Darstellung des makrozyklischen, polyfunktionellen Diterpens 6,17-Epoxylathyrol aus Samenöl von Euphorbia lathyris L.

Das Öl wird durch Extraktion der mit einem Fleischwolf zerklienerten Samen von E. lathyris L. mit peroxidfreiem Diäthyläther und anschließendes Abziehen des Lösungsmittels am Rotationsverdampfer gewonnen. Beim längeren Stehen des Samenöls bei 4°C kristallisiert daraus das farblose 6,17-Epoxylathyrol-3-phenylacetat-5,15-diacetat aus, das aus heißem Äthanol umkristallisiert wird, Schmp.: 199—200°C. 2 g 6,17-Epoxylathyrol-3-phenylacetat-5,15-diacetat werden in 80 ml Methanol gelöst und mit 100 ml 0,5 n methanolischer Kalilauge versetzt. Nach 3-stündigem Stehen unter Stickstoff bei Raumtemperatur stoppt man die Reaktion mit 6 ml Eisessig ab, engt am Rotationsverdampfer ein versetzt den Rückstand mit Methylenchlorid und saugt vom ausgefallenen Kaliumacetat ab. Das Reaktionsprodukt wird in 150 ml Methylenchlorid gelöst und dreimal mit je 100 ml 2 n Sodalösung ausgeschüttelt, um die freigewordene Phenylessigsäure zu entfernen. Man wäscht mit wenig Wasser alkalifrei, trocknet über Magnesiumsulfat und erhält nach Absaugen und Einengen 1150 mg 6,17-Epoxylathyrol (90% Ausbeute). Nach 3-maligem Umkristallisieren aus Äthanol/Wasser und Trocknen im Hochvakuum erhält man das makrozyklische polyfunktionelle Diterpen mit einem Schmp.: 204—207°C.

## Literatur

1. J. L. Hartwell, Plants used against Cancer. A. Survey. Lloydia *32,* 153—205 (1969); *34,* 250—252 (1971)

2. E. Hecker und R. Schmidt, Phorbolesters — the Irritants and Cocarcinogens of Croton tiglium L., Progr. Chem. Org. Nat. Products *31,* 375—467 (1974); E. Hecker Isolation and Characterization of the Cocarcinogenic Principles from Croton Oil in Methods in Cancer Research, Vol. VI, edit. by H. Busch, p. 439—484, 1971, Academic Press, New York-London.

3. E. Hecker, New Toxic, Irritants and Cocarcinogenic Diterpene Esters from Euphorbiaceae and from Thymelaeaceae, Pure appl. Chem. *49,* 1423—1431 (1977).

4. E. Hecker, Structure-Activity Relationships in Diterpene Esters Irritant and Cocarcinogenic to Mouse Skin, in Carcinogenesis, Vol. 2, Mechanisms of Tumor Promotion and Cocarcinogenesis, edit. by T. J. Slaga, A. Sivak und R. K. Boutwell, Raven Press, New York, 1978, pp. 11—48.

5. F. J. Evans und C. J. Soper, The Tigliane, Daphnane and Ingenane Diterpenes, Their Chemistry, Distribution and Biological Activities, Lloydia *41,* 193—233 (1978).

6. W. Adolf und E. Hecker, Diterpenoid Irritants and Cocarcinogens in Euphorbiaceae and Thymelaeceae: Structural Relationships in view of their Biogenesis, Israel J. Chem. *16,* 75—83 (1977).

7. E. Hecker, Aktuelle Probleme der Krebsentstehung, Z. Krebsforsch. *78,* 99—122 (1972).

8. E. Hecker, Definitions and Termology in Cancer (Tumor) Etiology An analysis aiming at proposals for a current internationally standardized terminology, Int. J. Cancer, 18, 122—129; vgl. auch Z. Krebsforsch., 86, 219—230; GANN, 67, 471—481; Bull, WHO, 54, 1—10, 1976, vgl. auch loc.cit. 6.

9. I. Berenblum und V. Lonai, The leukemogenic action of phorbol, Cancer Res. *30,* 2744—2748 (1970).

10. V. Armuth und I. Berenblum, Systemic promoting action of phorbol in liver and lung carcinogenesis in AKRmice, Cancer Res. *32,* 2259—2262 (1972).

11. V. Armuth, Leukemogenic action of phorbol in intact and thymectomized mice of different strains, Brit. J. Cancer *34,* 516—522 (1976).

12. V. Armuth und I. Berenblum, Promotion of mammary carcinogenesis and leukemogenic action by phorbol in virgin female Wistar rats, Cancer Res. *30,* 27 4—27 8 (1970).

13. D. Gericke, W. Kovac und E. Hecker, On a possible cocarcinogenic and immunosuppressive Activity of Phorbol in AKR-Mice, Z. Krebsforsch. 82, 183—189 (1974).

14. M. W. Baird und R. K. Boutwell, Tumor-promoting activity of phorbol and four diesters of phorbol in mouse skin. Cancer Res. *31,* 1074—1079 (1971).

15. V. Armuth und I. Berenblum, Phorbol as a possible systemic promoting agent for skin carcinogenesis. Z. Krebsforsch. *85,* 79—82 (1976).

16. S. M. Kupchan, C. W. Sigel, M. J. Matz, J. A. S. Renauld, R. C. Haltiwanger and R. F. Bryan, Jatrophone, a Novel Macrocyclic Diterpenoid Tumor Inhibitor from Jatropha gossypifolia, J. Amer. Chem. Soc. *92,* 4476 (1970).

17. S. M. Kupchan, C. W. Sigel, M. J. Matz, C. J. Gilmore and R. F. Bryan, Structure and Stereochemistry of Jatrophone, A Novel Macrocyclic Diterpenoid Tumor Inhibitor, J. Amer. Chem. Soc. *98,* 2295 (1976).

Die polyzyklischen Diterpene 1—4 und jeweils typische Diterpenderivate, die aus Euphorbiaceen und Thymelaeaceen isoliert wurden.

Daphnan (*1*)

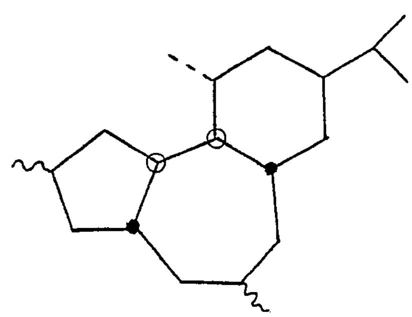

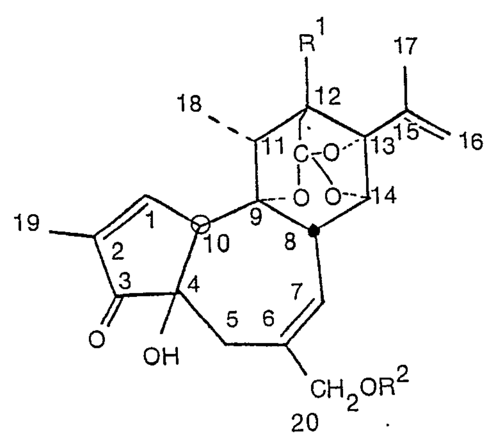

1a 9,13,14-ortho-Tetradekanoylresinifernonol: $R^1$ = Alkyl $R^2$ = H
1b Resiniferonol:

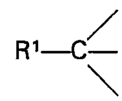 = 3H, $R^2$ = H

1c Resiniferatoxin: $R^1$ = $CH_2C_6H_5$, $R^2$ = $COCH_2C_6H_3$ (OH, $OCH_3$)
1d Simplexin: 5β-Hydroxy-6,7α-epoxy-resiniferonol-9,13,14-ortho-dekan-säureester

Tiglian (2)

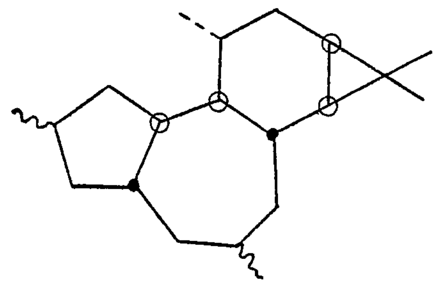

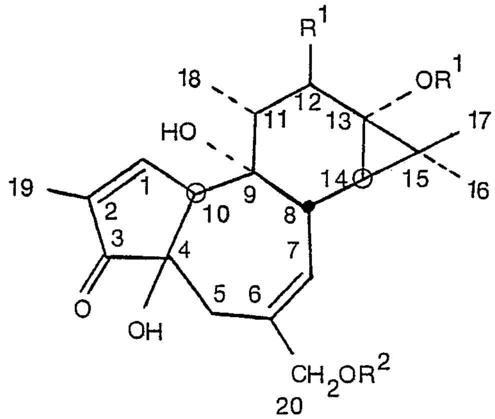

2a 13-O-Tetradekanoyl-12-desoxyphorbol: R′ = H, R′ = Tetradekanoyl, $R^2$ = H
2b 12-Desoxyphorbol: R′ = H, $R^1$ = $R^2$ = H
2c 12-O-Tetradekanoylphorbol-13-acetat (TPA): R′ = Tetradekanoyloxy, $R^1$ = $COCH_3$, $R^2$ = H
2d Phorbol: R′ = OH, $R^1$ = H, $R^2$ = H bzw. 12,13,20-triacetat, R′ = $OCOCH_3$, $R^1$ = $R^2$ = $COCH_3$
2e Kryptisches TPA: R′ = Tetradekanoyloxy, $R^1$ = $COCH_3$, $R^2$ = Acyl,
2f 4α-Phorbol: 2d entsprechend jew. mit 4α-Tiglianstruktur.

Ingenan (3)

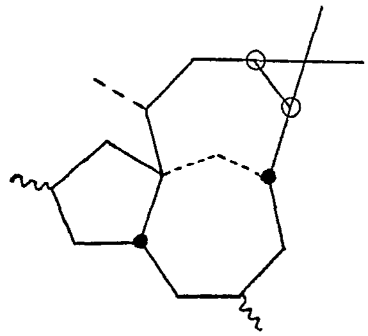

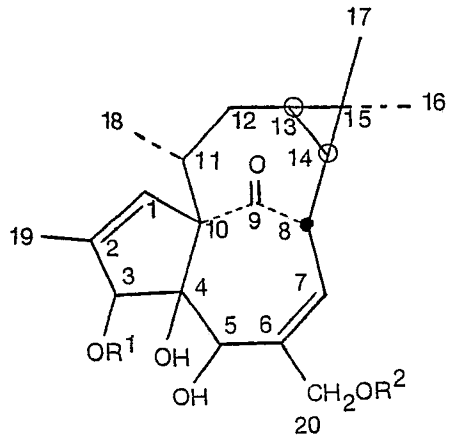

3a 3-Hexadekanoylingenol: $R^1$ = Hexadekanoyl, $R^2$ = H
3b Ingenol: $R^1 = R^2$ = H
3c Ingenol-3,20-dibenzoat: $R^1 = R^2 = COC_6H_5$
3d Kryptisches 3-Hexadekanoylingenol: $R^1$ = Hexadekanoyl, $R^2$ = Acyl
3e 16-Hydroxyingenol: vgl. 3b entsprechend

Lathyran (4)

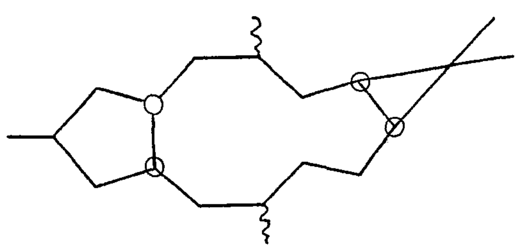

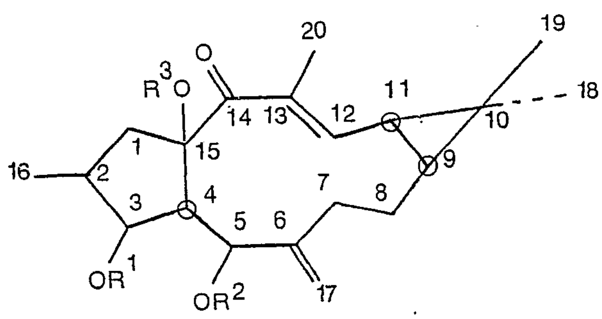

4a Diterpenester "$L_3$": $R^1 = R^2 = COCH_3$ $R^3 = COC_6H_5$
4b Ingol-12-acetat: mit 4β-Lathyranstruktur
4c Lathyrol: $R^1 - R^2 = R^3 = H$
4d 6,17-Epoxylathyrol-3-phenylacetat-5,15-diacetat

Anlage 3

Auf antineoplastische Wirkung getestete polyfunktionelle Diterpenester des Tiglian-, Daphnan- und Ingenantyps

| Substanz | Struktur-typ | Tumor-modell | Wirkung (T/C) | Ref. Nr. +) |
|---|---|---|---|---|
| Phorbol-12-tigliat-13-decanoat | 1 | P–388 | ≥125% (60–260 μg/kg) | 71 |
| Mezerein | 2 | P–388<br>L–1210 | ≥125% (50 μg/kg) | 69 |
| Gnididin | 2 | P–388 | ≥125% (20–100 μg/kg) | 70 |
| Gniditrin | ,, | ,, | ,, | ,, |
| Gnidicin | ,, | ,, | ,, | ,, |
| Gnidilatin | 2 | P–388 | 130–140% (20–80 μg/kg) | 72 |
| Gnidilatin-20-palmitat | ,, | ,, | ≥125% (20 μg/kg) | ,, |
| Gnidilatidin-20-palmitat | ,, | ,, | 170% (0,5 – 2 mg/kg) | ,, |
| Gnidimacrin | 2 | P–388 | 180% (12–16 μg/kg) | 73 |
| Gnidimacrin-20-palmitat | ,, | ,, | 190% (30–50 μg/kg) | ,, |
| Ingenol-3,20-dibenzoat | 3 | P–388 | ≥125% (130–360 μg/kg) | 71 |

+) alle Ziffern dieser Spalte beziehen sich auf das in Anlage 1, loc. cit. 4 enthaltene Literaturverzeichnis.

Anlage 4, Protokoll 1

♀ ♂ Fa.:

| | | | |
|---|---|---|---|
| ASSISTENT | : Bollow | TRANSPL.–DATUM | : 14.1.77 |
| VERSUCH NR. | : | THERAPIEBEGINN | : 17.1.77 |
| STAMM | : Swiss | BEHANDLUNGSTAGE | : 1x wöchen. |
| TUMOR | : Sarkom 180 | THERAPIEDAUER | : 3 Wochen |
| TRANSPL.–MODUS | : i.m. | VERSUCHSENDE | : 4.2.77 |
| TUMORGEW. BEI BEHANDL. BEG. | : 1,2 ϕ Gramm | TIERZAHL/SERIE | : 15 |

| SERIE Nr. | PRÄPARAT | EINZEL-DOSIS mg/Kg | GESAMT-DOSIS mg/Kg | GE-HEILT | † | ϕ ± Tumor Gewicht | ϕ Körpergew. Differenz |
|---|---|---|---|---|---|---|---|
| 1 | negative Kontrolle | — | — | — | — | 12,20<br>1,52 | –20,02% |
| 2 | T P A sc. | 0,5 | 1,5 | — | — | 5,12<br>0,68 | –3,13% |
| 3 | Phorbol sc. | 20 | 60 | — | — | 5,10<br>1,69 | –0,38% |

| SERIE Nr. | PRÄPARAT | EINZEL-DOSIS mg/Kg | GESAMT-DOSIS mg/Kg | GE-HEILT | † | ϕ ± Tumor Gewicht | ϕ Körpergew. Differenz |
|---|---|---|---|---|---|---|---|
| 4 | Simpexin sc. | 2 | 6 | — | — | 5,12<br>1,40 | –4,46% |
| 5 | Ihgenol-3,20-di-benzoat sc. | 5 | 15 | — | — | 5,72<br>1,70 | –3,42% |
| | *Anm. betr. Gruppe 4:* Bei allen Mäusen entstanden massive Hautentzündungen, wobie sich die Haare völlig ablösten. Die darunterliegende Hautfläche war hart und brüchig. | | | | | | |

Anlage 4, Protokoll 2

♀ ♂ Fa:

| | | | |
|---|---|---|---|
| ASSISTENT | : Bollow | TRANSPL.–DATUM | : 11.2.77 |
| VERSUCH NR. | : | THERAPIEBEGINN | : 14.2.77 |
| STAMM | : Swiss | BEHANDLUNGSTAGE | : |
| TUMOR | : Ehrlich-diploid | THERAPIEDAUER | : 3 Wochen |
| TRANSPL.–MODUS | : im | VERSUCHSENDE | : 7.3.77 |
| TUMORGEW. BEI. BEHANDL. BEG. | : 1,0 g ϕ | TIERZAHL/SERIE | : 15 |

| SERIE Nr. | PRÄPARAT | EINZEL-DOSIS mg/Kg | GESAMT-DOSIS mg/Kg | GE-HEILT | ✝ | ϕ ± Tumor Gewicht | ϕ Körpergew. Differenz |
|---|---|---|---|---|---|---|---|
| 1 | negative Kontrolle | – | – | – | – | +10,20<br>–1,27 | –11,25% |
| 2 | positive Kontrolle Endoxan sc. 1x wöch. | 90 | 270 | 5/15 | – | +3,35<br>–1,00 | –6,56% |
| 3 | T P A sc.<br>Mo. + Do. | 0,5 | 3,0 | – | – | +5,39<br>–1,75 | –2,32% |
| 4 | Simplexin Sc.<br>Mo. + Do. | 2 | 12 | 2/15 | 3/15 | +4,15/12<br>–2,20/12 | – |

| SERIE Nr. | PRÄPARAT | EINZEL-DOSIS mg/Kg | GESAMT-DOSIS mg/Kg | GE-HEILT | † | ϕ ± Tumor Gewicht | ϕ Körpergew. Differenz |
|---|---|---|---|---|---|---|---|
| 5 | Ingenol-3,20-dibenzoat sc. Mo. + Do. | 2,5 | 15,0 | – | – | +4,41<br>–1,37 | –3,44% |
| 6 | Resiniferatoxin sc. Mo. + Do. | 0,1 | 0,6 | – | – | +6,22<br>–2,66 | –4,55% |
| 7 | Phorbol oral Mo. + Mi. + Fr. | 40 | 360 | 2/15 | – | +6,00<br>–3,40 | –4,12% |
| 8 | Phorbol sc. Mo. + Mi. + Fr. | 40 | 360 | 2/15 | – | +5,05<br>–3,52 | –3,98% |
| 9 | Phorbol sc. Mo. + Do. | 40 | 240 | 2/15 | – | +4,30<br>–1,89 | –2,96% |
| 10 | Phorbol i.v. Mo. + Do. | 40 | 240 | 3/15 | – | +4,90<br>–2,61 | –1,10% |

Anlage 5
♀♂ Fa.:

ASSISTENT : Merkel
VERSUCH NR. : 13/1–19
STAMM : Swiss
TUMOR : Retothel-Sarkom
TRANSPL.–MODUS : i.m.
TUMORGEW. BEI BEHANDL. BEG. : ca. 1,5 g

TRANSPL.–DATUM : 25.4.77
THERAPIEBEGINN : 28.4.77
BEHANDLUNGSTAGE : 7
THERAPIEDAUER : 4 Wochen
VERSUCHSENDE : 24.5.77
TIERZAHL/SERIE : 15

| SERIE Nr. | PRÄPARAT | EINZEL-DOSIS mg/Kg | GESAMT-DOSIS mg/Kg | GE-HEILT | † | ⌀ ± Tumor Gewicht | ⌀ Körpergew. Differenz |
|---|---|---|---|---|---|---|---|
| 1 | negative Kontrolle | — | — | — | — | 10,6<br>2,0 | 33,2<br>–11,7% |
| 2 | positive Kontrolle | 60 | 240 | — | — | 4,1<br>1,1 | 31,0<br>– 3,8% |
| 3 | Endoxan sc. | 90 | 360 | — | — | 2,9<br>1,1 | 31,8<br>– 2,5% |
| 4 | 4α-Phorbol-sc. nach 72h Wiederholung | 80 | 400 | — | — | 3,7<br>2,5 | 33,8<br>+2,6% |

| SERIE Nr. | PRÄPARAT | EINZEL-DOSIS mg/Kg | GESAMT-DOSIS mg/Kg | GE-HEILT | † | ϕ ± Tumor Gewicht | ϕ Körpergew. Differenz |
|---|---|---|---|---|---|---|---|
| 5 | Phorbol sc. nach 72 h Wiederholung | 80 | 480 | — | — | 2,6<br>1,0 | 30,6<br>+ 5,8% |
| 6 | 16-Hydroxy-Ingenol sc. nach 72 h Wiederholung | 80 | 560 | — | — | 2,5<br>1,0 | 31,9<br>+ 5,0% |
| | *Anmerkung zu den Gruppen 4 un 5:* Aus Substanzmangel wurde bei Versuch Nr. 4 in der letzten Woche nicht mehr behandelt und bei Versuch Nr. 5 auf die letzte Injektion in der letzten Woche verzichtet. | | | | | | |

Anlage 6, Protokoll 1
♀♂ Fa.:

ASSISTENT : You./De.
VERSUCH NR. : 4/78
STAMM : $D_2B_6F_1$
TUMOR : P 388
TRANSPL.–MODUS : ip.
TUMORGEW. BEI BEHANDL. BEG. : ––

TRANSPL.–DATUM : 27.2.78
THERAPIEBEGINN : 28.2.78
BEHANDLUNGSTAGE : 1+4x woch.
THERAPIEDAUER : 1 Woche
VERSUCHSENDE : 17.4.78
TIERZAHL/SERIE : 15

| SERIE Nr. | PRÄPARAT | EINZEL-DOSIS mg/Kg | GESAMT-DOSIS mg/Kg | GE-HEILT | † | ILS % | Ø Überlebenszeit Median |
|---|---|---|---|---|---|---|---|
| 1 | negative Kontrolle | – | – | – | 15/15 | – | 10–*14,00*–16 |
| 2 | positive Kontrolle Endoxan sc. | 40 | 40 | 7/15 | 8/15 | 90,93 | 16–*23,87*–42 |
| 3 | positive Kontrolle Endoxan sc. | 30 | 30 | 9/15 | 6/15 | 101,42 | 7–*25,50*–51 |
| 4 | positive Kontrolle Endoxan sc. | 120 | 120 | 11/15 | 4/15 | 91,90 | 14–*18,25*–29 |
| 5 | Phorbol sc. | 100 | 100 | 12/15 | 3/15 | 96,65 | 14–*17,66*–21 |
| 8 | Phorbol sc. an vier Tagen | 4×100 | 400 | 9/15 | 6/15 | 85,71 | 12–*20,00* 51 |

Anlage 6, Protokoll 2
♀♂ Fa.:

ASSISTENT : You./De.
VERSUCH NR. : 4/78
STAMM
TUMOR :
TRANSPL.–MODUS :
TUMORGEW. BEI BEHANDL. BEG. :

TRANSPL.–DATUM :
THERAPIEBEGINN :
BEHANDLUNGSTAGE :
THERAPIEDAUER :
VERSUCHSENDE :
TIERZAHL/SERIE :

| SERIE Nr. | PRÄPARAT | EINZEL-DOSIS mg/Kg | GESAMT-DOSIS mg/Kg | GE-HEILT | † | ILS % | ∅ Überlebenszeit Median |
|---|---|---|---|---|---|---|---|
| 9 | 4α-Phorbol sc. | 100 | 100 | 10/15 | 5/15 | 89,04 | 12–19,40–37 |
| 12 | 12-Desoxy-phorbol sc. | 100 | 100 | 8/15 | 7/15 | 62,85 | 8–14,57–37 |
| 13 | 12-Desoxy-phorbol sc. an vier Tagen | 4 × 100 | 400 | 7/15 | 8/15 | 59,47 | 11–33,25–15 |
| 14 | Phorbol-12,13-20-triacetat sc. | 0,25 | 0,25 | 3/15 | 12/15 | 14,28 | 9–12,50–14 |
| 18 | Lathyrol sc. | 150 | 150 | 5/15 | 10/15 | 120,17 | 13–31,3–40 |

Anlage 5, Protokoll 3

♀♂ Fa.:

ASSISTENT : You./De.
VERSUCH NR. : 4/78
STAMM :
TUMOR :
TRANSPL.–MODUS :
TUMORGEW. BEI BEHANDL. BEG. :

TRANSPL.–DATUM :
THERAPIEBEGINN :
BEHANDLUNGSTAGE ::
THERAPIEDAUER :
VERSUCHSENDE :
TIERZAHL/SERIE :

| SERIE Nr. | PRÄPARAT | EINZEL-DOSIS mg/Kg | GESAMT-DOSIS mg/Kg | GE-HEILT | † | ILS % | ϕ Überlebenszeit Median |
|---|---|---|---|---|---|---|---|
| 19 | Lathyrol sc. an vier Tagen | 4×75 | 300 | 10/15 | 5/15 | 71,42 | 10–*12,00*–13 |
| 20 | Ingenol-12-acetate sc. | 200 | 200 | 9/15 | 6/15 | 94,74 | 9–*23,16*–50 |
| 21 | 6,17-Epoxylathyrol-3-Phenylacetat-5,15-diacetat | 400 | 400 | 8/15 | 7/15 | 58,55 | 13–*13,28*–15 |
| 22 | Ingenol-12-acetat sc. | 400 | 400 | 7/15 | 8/15 | 106,17 | 11–*23,87*–37 |

| SERIE Nr. | PRÄPARAT | EINZEL-DOSIS mg/Kg | GESAMT-DOSIS mg/Kg | GE-HEILT | † | ILS % | Überlebenszeit ⌀ Median |
|---|---|---|---|---|---|---|---|
| 23 | Phorbol-12,13-20-tribenzoat sc. | 100 | 100 | 9/15 | 6/15 | 76,65 | 11–*16,83*–28 |
| 26 | Phorbol-12,13,20-tribenzoat sc. an 4 Tagen | 4 × 100 | 400 | 8/15 | 7/15 | 55,21 | 10–*12,28*–14 |

Anlage 6, Protokoll 4
♀♂ Fa.:

ASSISTENT : You./De.
VERSUCH NR. : 4/78
STAMM :
TUMOR :
TRANSPL.–MODUS :
TUMORGEW. BEI BEHANDL. BEG. :

TRANSPL.–DATUM :
THERAPIEBEGINN :
BEHANDLUNGSTAGE :
THERAPIEDAUER :
VERSUCHSENDE :
TIERZAHL/SERIE :

| SERIE Nr. | PRÄPARAT | EINZEL-DOSIS mg/kg | GESAMT-DOSIS mg/Kg | GE-HEILT | † | ILS % | ⌀ Überlebenszeit Median |
|---|---|---|---|---|---|---|---|
| 27 | Myrsinol-ester-gemisch sc. | 25 | 25 | 5/15 | 10/15 | 49,04 | 11–*16,30*–5 |
| 28 | | 50 | 50 | 6/15 | 9/15 | 41,42 | 12–*13*–15 |
| 29 | | 4×25 | 100 | 10/15 | 5/15 | 71,42 | 10–*12*–14 |
| 30 | 4α-Phorbol-12,13,20-triacetat sc. | 200 | 200 | 8/15 | 7/15 | 77,61 | 14–*19*–44 |
| 31 | | 400 | 400 | 11/15 | 4/15 | 90,47 | 12–*17,50*–32 |

**Patentansprüche**

1. Antineoplastische Mittel, gekennzeichnet durch einen Gehalt an nicht oder nur geringfügig irritierenden und nicht oder nur geringfügig promovierenden Alkoholen, Estern, Äthern oder Acetalen polyfunktioneller Diterpene des Tiglian-, Daphnan-, Ingenan- oder Lathryan- typs als Wirksubstanz neben üblichen pharmazeutischen Hilfsstoffen und gegebenenfalls zusätzlichen aktiven Stoffen, wobei die Ester solche mit nicht substituierten aliphatischen oder aromatische Säuren bis zu 20 C-Atomen und die Äther bzw. Acetale solche mit nicht substituierten Alkoholen bzw. Carbonylverbindungen bis zu 20 C-Atomen sind.

2. Mittel nach Anspruch 1, gekennzeichnet durch einen Gehalt an sauren Carbonsäureestern oder deren Salzen mit pharmakologisch gut verträglichen Basen sowie basischen Estern von Aminosäuren oder Peptiden oder deren Ammoniumsalzen mit pharmakologisch gut verträglichen Säuren.

3. Verfahren zur Herstellung von antineoplastischen Mitteln nach Anspruch 1 oder 2, dadurch gekennzeichnet, daß man die entsprechenden polyfunktionellen Diterpene mit nichtsubstituierten aliphatischen oder aromatischen Carbonsäuren mit bis zu 20 C-Atomen oder nicht substituierten Alkohol- oder Carbonylverbindungen mit bis zu 20 C-Atomen in an sich bekannter Weise in dei entsprechenden nicht oder nur geringfügig irritierenden und nicht oder nur geringfügig promovierenden Ester, Äther oder Acetale überführt und so erhaltene saure oder basische Ester in an sich bekannter Weise mit pharmakologisch verträglichen Basen oder Säuren in Salze überführt und die so erhaltenen Verbindungen zusammen mit üblichen pharmazeutischen Hilfsmitteln zu antineoplastischen Mitteln konfektioniert.

4. Verfahren nach Anspruch 3, dadurch gekennzeichnet, daß man die entsprechenden polyfunktionellen Diterpene zur Herstellung saurer Ester mit Dicarbonsäuren oder Anhydriden davon, umsetzt.

5. Verfahren nach Anspruch 4, dadurch gekennzeichnet, daß man als Anhydride von Dicarbonsäuren solche der Bernsteinsäure, Glutarsäure oder Phthalsäure verwendet.

6. Verfahren nach Anspruch 4 oder 5, dadurch gekennzeichnet, dass man die sauren Ester mit pharmakologisch gut verträglichen Aminen zum entsprechenden Salz umsetzt.

7. Verfahren nach Anspruch 6, dadurch gekennzeichnet, daß man als Amine Aminoalkohole oder Morpholin verwendet.

8. Verfahren nach Anspruch 3, dadurch gekennzeichnet, daß man die entsprechenden polyfunktionellen Diterpene mit Aminosäuren oder mit Peptiden in an sich bekannter Weise zu basischen Estern der Aminosäuren oder Peptide umsetzt.

9. Verfahren nach Anspruch 8, dadurch gekennzeichnet, daß man als Aminosäuren oder Peptide geeignet geschützte und aktivierte Derivate davon einsetzt.

10. Verfahren nach Anspruch 8 oder 9, dadurch gekennzeichnet, daß man die basischen Ester mit Aminosäuren oder Peptiden mit pharmakologisch gut verträglichen anorganischen oder organischen Säuren in die entsprechenden Ammoniumsalze überführt.

**Revendications**

1. Produits antinéoplasiques, caractérisés par une teneur en alcools, esters, éthers ou acétals, pas ou seulement peu irritants et pas ou seulement peu promoteurs, de diterpènes polyfonctionnels du type tigliane, daphnane, ingénage ou lathyrane en tant que principe actif, outre des produits auxiliaires pharmaceutiques habituels et éventuellement des substances actives supplémentaires, où les esters sont des esters d'acides aliphatiques ou aromatiques, non substitués, avec jusqu'à 20 atomes de carbone, et les éthers ou acétals sont des éthers ou acétals d'alcools ou composés carbonyliques non-substitués avec jusqu'à 20 atomes de carbone.

2. Produits selon la revendication 1, caractérisés par une teneur en esters acides d'acides carboxyliques ou en leurs sels avec des basis pharmacologiquement bien compatibles ainsi qu'en esters basiques d'acides aminés ou de peptides ou leurs sels d'ammonium avec des acides pharmacologiquement bien compatibles.

3. Procédé pour la préparation de produits antineoplasiques selon la revendication 1 ou 2, caractérisé en ce qu'on convertit les diterpènes polyfonctionnels correspondants, avec des acides carboxyliques aliphatiques ou aromatiques non-substitués avec jusqu'à 20 atomes de carbon ou des composés alcool ou carbonyliques non-substitués avec jusqu'à 20 atomes de carbone, d'une manière connue en soi en les esters, éthers ou acétals correspondants, pas ou seulement peu irritants, et pas ou seulement peu promoteurs, et qu'on convertit en sels, d'une manière connue en soi, les esters acides ou basiques ainsi obtenus avec des bases ou acides pharmacologiquement compatibles, et qu'on transforme en produits antinéoplasiques les compositions ainsi obtenues, ensemble avec des produits auxiliaires pharmaceutiques habituels.

4. Procédé selon la revendication 3, caractérisé en ce qu'on fait réagir les diterpènes polyfonctionnels correspondants pour la préparation d'esters acides avec des acides dicarboxyliques ou leurs anhydrides.

5. Procédé selon la revendication 4, caractérisé en ce qu'on utilise en tant qu'anhydrides d'acides dicarboxyliques ceux de l'acide succinique, de l'acide glutarique ou de l'acide phtalique.

6. Procédé selon la revendication 4 ou 5, caractérisé en ce qu'on fait réagir en le sel

correspondant les esters acides avec des amines pharmacologiquement bien compatibles.

7. Procédé selon la revendication 6, caractérisé en ce qu'on utilise en tant qu'amines des aminalcools ou la morpholine.

8. Procédé selon la revendication 3, caractérisé en ce qu'on fait réagir les diterpènes polyfonctionnels correspondants avec des acides aminés ou avec des peptides, d'une manière connue en soi, pour donner des esters basiques d'acides aminés ou de peptides.

9. Procédé selon la revendication 8, caractérisé en ce qu'on utilise en tant qu'acides aminés ou peptides leurs dérivés convenablement protégés et activés.

10. Procédé selon la revendication 8 ou 9, caractérisé en ce qu'on convertit les esters basiques d'acides aminés ou peptides avec des acides organiques ou inorganiques pharmacologiquement bien compatibles, en les sels d'ammonium correspondants.

**Claims**

1. Antineoplastic agent, characterised in that it contains non-irritating, or only slightly irritating, and non-promoting, or only slightly promoting, alcohols, esters, ethers or acetals of polyfunctional diterpenes of the tiglian, daphnan, ingenan or lathyran type as the active substance, in addition to customary pharmaceutical auxiliaries and, if appropriate, additional active substances, the esters being those with unsubstituted aliphatic or aromatic acids of up to 20 C atoms and the ethers and acetals being those with unsubstituted alcohols or carbonyl compounds of up to 20 C atoms.

2. Agents according to Claim 1, characterised in that they contain acid carboxylic acid esters or salts thereof with particularly pharmacologically acceptable bases and basic esters of aminoacids or peptides or ammonium salts thereof with particularly pharmacologically acceptable acids.

3. Process for the preparation of antineoplastic agents according to Claim 1 or 2, characterised in that the corresponding polyfunctional diterpenes are converted into the corresponding non-irritating, or only slightly irritating, and non-promoting, or only slightly promoting esters, ethers or acetals in a manner which is known per se with unsubstituted aliphatic or aromatic carboxylic acids with up to 20 C atoms or unsubstituted alcohol or carbonyl compounds with up to 20 C atoms, and the acid or basic esters thus obtained are converted into salts in a manner which is known per se with pharmacologically acceptable bases or acids, and the compounds thus obtained are formulated to antineoplastic agents together with customary pharmaceutical auxiliaries.

4. Process according to Claim 3, characterised in that the corresponding polyfunctional diterpenes are reacted with dicarboxylic acids or anhydrides thereof for the preparation of acid esters.

5. Process according to Claim 4, characterised in that the anhydrides of dicarboxylic acids used are those of succinic acid, glutaric acid or phthalic acid.

6. Process according to Claim 4 or 5, characterised in that the acid esters are reacted with particularly pharmacologically acceptable amines to give the corresponding salt.

7. Process according to Claim 6, characterised in that aminoalcohols or morpholine are used as the amines.

8. Process according to Claim 3, characterised in that the corresponding polyfunctional diterpenes are reacted with aminoacids or with peptides in a manner which is known per se to give basic esters of the aminoacids or peptides.

9. Process according to Claim 8, characterised in that the aminoacids or peptides used are suitably protected and activated derivatives thereof.

10. Process according to Claim 8 or 9, characterised in that the basic esters with aminoacids or peptides are converted into the corresponding ammonium salts with particularly pharmacologically acceptable inorganic or organic acids.